# EUROPEAN PATENT SPECIFICATION

(11) **EP 0 327 743 B1**
(45) Date of publication and mention of the grant of the patent: **20.01.1993**
(21) Application number: 88301160.3
(22) Date of filing: 11.02.1988
(51) Int. Cl.: C07C 2/36, C07F 9/50

(54) **Procedure for manufacturing short-chain linear alpha- olefines from ethylene**
Verfahren zur Herstellung von Alpha-Olefinen mit kurzer Kette aus Äthylen
Procédé pour la préparation d'alpha-oléfines à chaîne courte à partir d'éthylène

(43) Date of publication of application: 16.08.1989
(73) Proprietor: NESTE OY, SF-02150 Espoo 15 (FI)
(72) Inventor: Koskimies, Salme, SF-00930 Helsinki (FI); Rantanen, Leena, SF-06450 Porvoo (FI); Kivi, Jouni, SF-02150 Espoo (FI); Haime, Erkki, SF-00660 Helsinki (FI)
(74) Representative: Lamb, John Baxter

(56) References cited:
- EP-A- 0 014 239
- US-A- 3 686 351
- ORGANOMETALLICS, vol. 2, no. 5, 1983, pages 594-597, American Chemical Society; M. PEUCKERT et al.: "A new nickel complex for the oligomerization of ethylene"

## Description

The present invention concerns a procedure for manufacturing short-chain linear α-olefines, in particular 1-butylene, from ethylene.

These α-olefines have a number of special practical applications, depending on the length of the olefine's chain. For instance, C₁₂-C₂₀ α-olefines are used in the manufacturing of corresponding biodegradable washing materials of sulphonate and ethoxylate type. Alcohols prepared fro: C₈-C₁₂ α-olefines have significant use especially as softener alcohols. Furthermore, the use of C₄-C₆ α-olefines, particularly as a copolymer together with polyethylene, has increased in recent years, polyethylene brands made by combination catalysis (e.g. LLDPE) displacing traditional high pressure processes (LDPE) in many areas of practical application. Moreover, t-decylene is used in continuously increasing quantities in the manufacture of poly-α-olefin-type synthetic lubricants.

In industrially utilized manufacturing methods, preparation of 1-butylene is effected either by performing separation of butylene isomers from the C₄ flow obtained on cracking or by distilling the 1-butylene apart e.g. from the olefine mixture produced in the manufacturing of α-olefines.

When manufacturing 1-butylene from ethylene, one usually also obtains other oligomerizing products of ethylene in abundance, e.g. 2-butylene and other oligomers of varying length.

It is well-known that many catalysts can be used to oligomerize ethylene to yield olefines which have higher molecular weight. In this publication oligomerizing is understood to mean ethylene oligomerizing to become dimer, trimer, tetramer, etc., the aim being an oligomer with maximal linearity and having a double bond in a position.

It is a typical feature of olefine-oligomerizing reactions that the rate of reaction and the product distribution are decisively dependent on the catalyst type, especially on its exact chemical structure, and on the reaction conditions applied. For instance, the drawback of well-known aluminium alkyl or aluminium alkyl/titanium halide catalysts of "Ziegler" or "Ziegler-Natta" type is their high reactivity and strong pyrophory although the reaction conditions (pressure, temperature) may be somewhat lower than those in the procedure of the present invention.

The previously known catalysts of nickel phosphine type which are technically utilized in ethylene oligomerizing reactions, on the other hand, operate in such a way that mainly the above-mentioned C₁₂-C₂₀ α-olefines are obtained as reaction products. Differing from these, the invention concerns a procedure which enables the ethylene oligomerizing reaction to be controlled so that the products which are obtained are specifically linear α-olefines with lover boiling point, used as starting substances for plastics and/or synthetic lubricants.

By the procedure constituting the object of the invention particularly 1-butylene can be manufactured selectively and with high conversion (60-70%). The catalyst employed in the dimerizing procedure differs from any other known nickel phosphine catalyst used in oligomerizing ethylene in respect of the promotors used to modify the catalyst.

The procedure constituting the object of the invention also differs from other nickel phosphine-catalytic procedures particularly in that the catalysts which are employed have been prepared either by synthesizing a new phosphine ligand and/or changing the reactivity of the previously known phosphine catalyst e.g. with the aid of amines in a way causing the reaction to be directed towards short-chain linear α-olefines.

According to the invention there is provided a process for manufacturing 1-butylene by selectively dimerizing ethylene, using as a catalyst a nickel chelate in which the ligand is diphenyl(carboxymethyl)-phosphine, diphenyl(2-carboxyethyl)-phosphine or their alkali metal salts or an alkali metal salt of diphenyl(2-hydroxyethyl)phosphine, in the presence of an amine derivative.

Diphenyl(2-carboxyethyl)phosphine is also known under the names (beta-carboxyethyl)diphenylphosphine and diphenylphosphinopropionic acid and diphenyl(carboxymethyl)phosphine, under the name diphenylphosphinoacetic acid.

It is thus understood that in the present procedure for catalyst is used nickel diphenylphosphinopropionic acid chelate or nickel diphenylphosphinoacetic acid chelate or nickel diphenyl(2-hydroxyethyl)-phosphinochelate, together with an amine promotor.

The alkali metal salts of diphenylphosphinoacetic acid or propionic acid can be prepared e.g. by treating said acids with an alkali metal hydroxide, an oxide or a free metal. For diphenyl(2-hydroxyethyl)phosphine ligand, it is simplest to use a straight alkali metal salt prepared by reaction between triphenylphosphine, potassium and 2-chloroethanol.

Particular significance from the viewpoint of the present invention attaches to the synergy of the structures of the phosphine ligand molecule and the amine. Amine modification not only changes the reactivity of the catalyst so that the formation of linear α-olefines havinig a longer chain is reduced: it also significantly increases the total conversion of ethylene to α-olefine and thus also acts as promotor.

The use of amine propotors in the procedure constituting the object of the invention together with nickel diphenylphosphinopropionic acid chelate or nickel diphenylphosphinoacetic acid chelate or nickel diphenyl(2-hydroxyethyl)phosphine chelate significantly increases the conversion of ethylene particularly to butylene and also increases the 1-butylene selectivity. It is particularly significant in that connection that with increasing conversion, when amine promotors are used, the proportion of longer-chain olefines does not increase in the same proportion: said promotor accelerates above all the dimerizing reaction and, correspondingly, inhibits the formation of longer-chain olefines produced from ethylene in oligomerization.

We shall in the following present, in greater detail, the procedure of the invention and its advantageous embodiments.

The invention especially concerns those procedures in which nickel diphenylphosphinopropionic acid chelate, nickel diphenylphosphinoacetic acid chelate or nickel diphenyl(2-hydroxyethyl)phosphine chelate together with a promotor is dissolved at 10 to 50^{o}C in a polar organic solvent preferably containing oxygen, nitrogen or sulphur atoms (polyols, glycols, glycolethers, ethers, amides, cyano compounds, sulphonyl derivatives, etc.). Solvents which are suitable in view of the reaction are also non-polar organic solvents, such as aliphatic and aromatic hydrocarbons and their chlorine, bromine or fluorine derivatives.

The recommendable ethylene pressure during reaction is 0.069 to 35 MPa, preferably 2.8 to 10 MPa. A suitable reaction temperature is 50 to 200^{o}C, preferably however 60 to 130^{o}C, and reaction time, 0.5 to 50 hrs, preferably 3 to 30 hrs. The molar proportions regarding nickel salt, ligand, reducing agent and promotor which are to be recommended are 1-5:1:1-10:1-5, preferably 1.5-3:1:3-6:1-7.

For nickel derivative may be used nickel chloride, bromide or iodide, while however it is particularly favourable in view of the reaction to use nickel acetate, of which the acetate part then also serves as reaction promotor.

Activation of the reaction is accomplished with the aid of a reducing agent. Various borohydride salts, preferably alkali metal borohydrides, are appropriate in such use.

Suitable compounds for use as amine-type promotors in the reaction are primary, secondary and tertiary both aromatic, aliphatic and cyclic monoamines and diamines, e.g. butylamine, triethylamine, N,N,N',N'-tetramethylethyldiamine, N,N-dimethylpiperazine, etc.

As regards the diphenylphosphinopropionic acid ligand or the diphenylphosphinoacetic acid ligand, they may be used as they are, or the corresponding alkali metal salt may be prepared, e.g. by treating them with an alkali metal hydroxide, an oxide or a free metal. For diphenyl(2-hydroxyethyl)phosphine ligand, it is simplest to use an alkali metal salt directly prepared by means of a reaction between triphenylphosphine, potassium and 2-chloroethanol.

It is thus essential in the procedure of the invention, and differing from other methods presently known, that by using, for catalyst, nickel phosphinoacetic acid chelate, nickel phosphinopropionic acid chelate or nickel (2-hydroxyethyl)phosphine chelate in combination with an amine derivative, ethylene can be oligomerized into short-chain linear α-olefines, and that the use of an amine derivative not only inhibits the formation of longer-chain α-olefines but also acts as an efficient promotor, increasing the ethylene conversion.

It is another advantage gained with the aid of the procedure of the Invention that purification of the 1-butylene from polymerizing quality to required level can be carried out with ease, in the case of a reaction product prepared by the procedure of the invention (Table 3), by distillation. This is because iso-butylene, which is harmful from the viewpoint of polymerization, is not formed at all, while 2-butylene is inert in any coordination-catalytic polymerizing system. This implies that difficult separation of butylene isomers is avoided.

The invention may be further more closely illustrated by the aid of the examples now following.

### Examples 1-3:-

Catalyst was prepared in pressurized reactors in a nitrogen atmosphere by adding diphenylphosphinopropionic acid, nickel chloride and 2N aqueous KOH solution to 125 ml of butylene diol. The reactor was pressurized with ethylene to 3.45 MPa, and the mix was agitated during 15 min., at 20-40oC. Reducing agent solution (NaBH₄ dissolved in 5 ml of N,N-dithylacetamide) was added in an N₂ atmosphere, and the pressure was raised with ethylene to 5.2 MPa. After 15 min. mixing, the temperature in the reactor was raised to 100^{o}C and thereafter kept constant as long as there was reaction. The reactor was cooled, and the gases, liquids and solids that had been formed were analyzed. The quantities of starting materials and products, and the conditions, are stated in Table 1.

### Examples 4-5:-

The same experimental arrangements were made as in Examples 1-3, except that nickel acetate was used for starting material instead of nickel chloride in preparing the catalyst. The conditions in the experiment, and the product quantity, are stated in Table 1. The product structure analysis obtained in Example 5 is given in Table 3.

### Examples 6-7:-

Arrangements for the experiment were made as in Examples 1-3, except that at the catalyst adding stage an amino derivative (n-butylamine or N,N-dimethylpiperazine) was added to the catalyst solution. The results are given in Table 1.

### Examples 8 and 9:-

Arrangements as in Examples 1-3, except that nickel acetate was used instead of nickel chloride and N,N-dimethylpiperazine was added for extra promotor. The results are given in Table 1.

### Examples 10-15:-

Arrangements as in Examples 1-3, except that calcium salt of diphenyl(2-hydroxyethyl)phosphine was used for ligand as such, without extra KOH addition. Promotors, acetate and/or amino (N.N-dimethylpiperazine) were added similarly as in Examples 4-9. The results are given in Table 2.

### Example 16:

The experiment was carried out as in Example 1, except that diphenylphosphinoacetic acid was used instead of diphenylphosphinopropionic acid, a temperature between 70 and 75^{o}C instead of 10^{o}C. The quantities of starting materials and products, and the conditions, are stated in Table 4.

### Example 17:

The experiment was carried out similarly as Example 16, except that 2N,N-dimethylpiperazine were also added to 125 ml butane diol. The results are given in Table 4.

### Examples 18-21:-

Experimental arrangements as in Examples 1 and 17, except that potassium salt of diphenyl(2-hydroxyethylene)phosphine was used for ligand, chloride or acetate for nickel salt and N,N-dimethylpiperazine for amine (Examples 4 and 6), and 90-100^{o}C for reaction temperature. The results are given in Table 4.

### Examples 22-25:-

Arrangements as In Examples 1 and 17, except that diphenylphosphinopropionic acid was used for ligand, chloride or acetate for nickel salt, and either butylamine (Experiment 23) or N,N-dimethylpiperazine (Examples 24 and 25) for amide. The reaction temperature which was applied was 90-100^{o}C. The results are given in Table 4.

The exact hydrocarbon structure distribution found for all products prepared in Examples 16-25 is stated in Table 5.

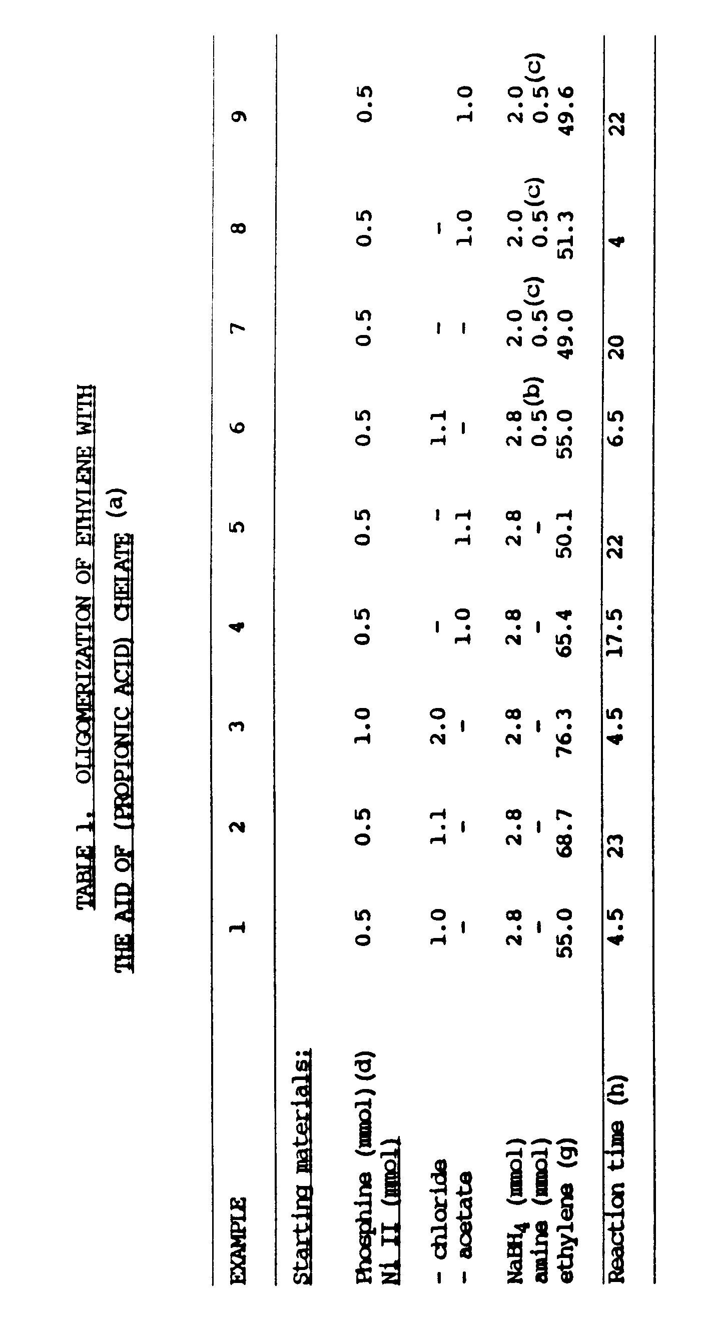

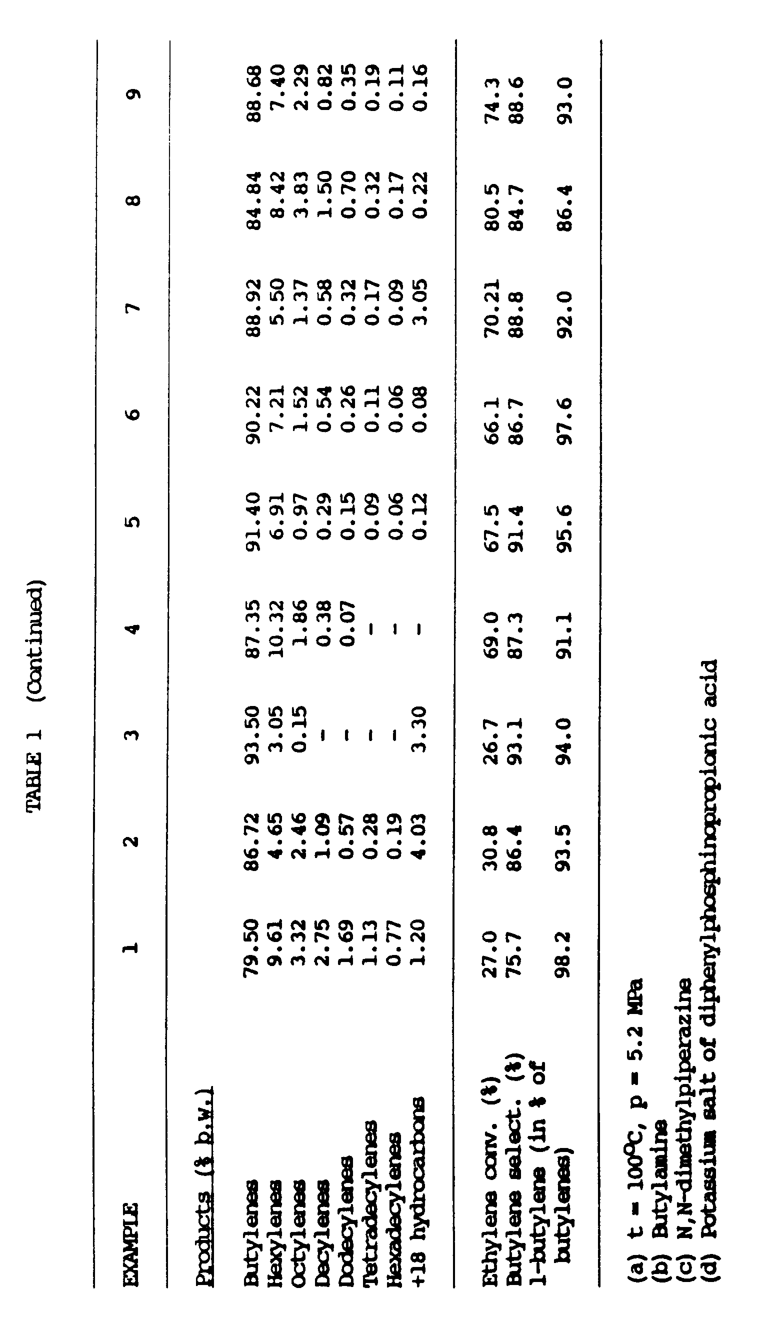

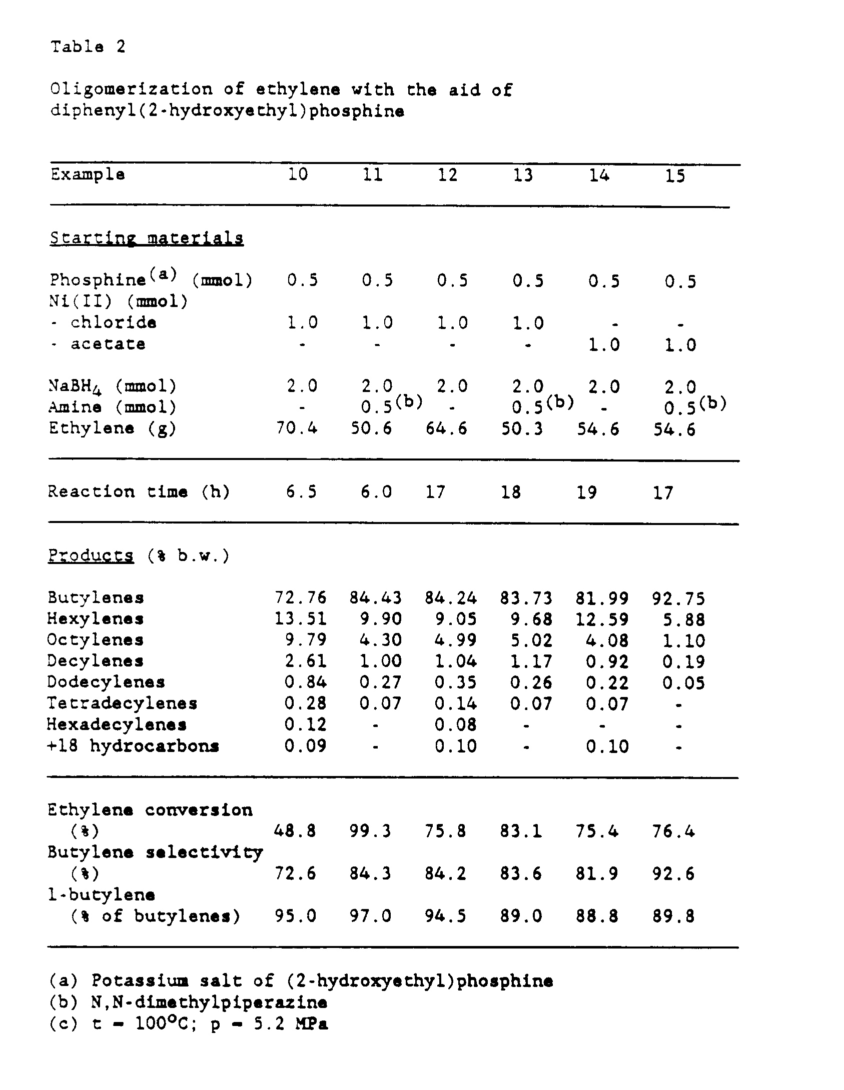

**Table 3**

| Typical product structure distribution in a gas sample as obtained in the ethylene oligomerizing reaction (Example 5). | |
|---|---|
| | % by weight |
| 2 Ethylene | 33.1 |
| 7 1-butylene | 60.1 |
| 9 n-butane | 0.12 |
| 10 Tr-2-butylene | 1.2 |
| 12 Cis-2-butylene | 1.5 |
| 18 2-Et-1-butylene | 0.4 |
| 33 3-Me-1-pentene | 0.02 |
| 43 1-hexylene | 3.0 |
| 45 Tr-3-hexylene | 0.1 |
| 46 Cis-3-hexylene | 0.1 |
| 105 C8H16 olef. | 0.04 |
| 111 1-octylene | 0.1 |

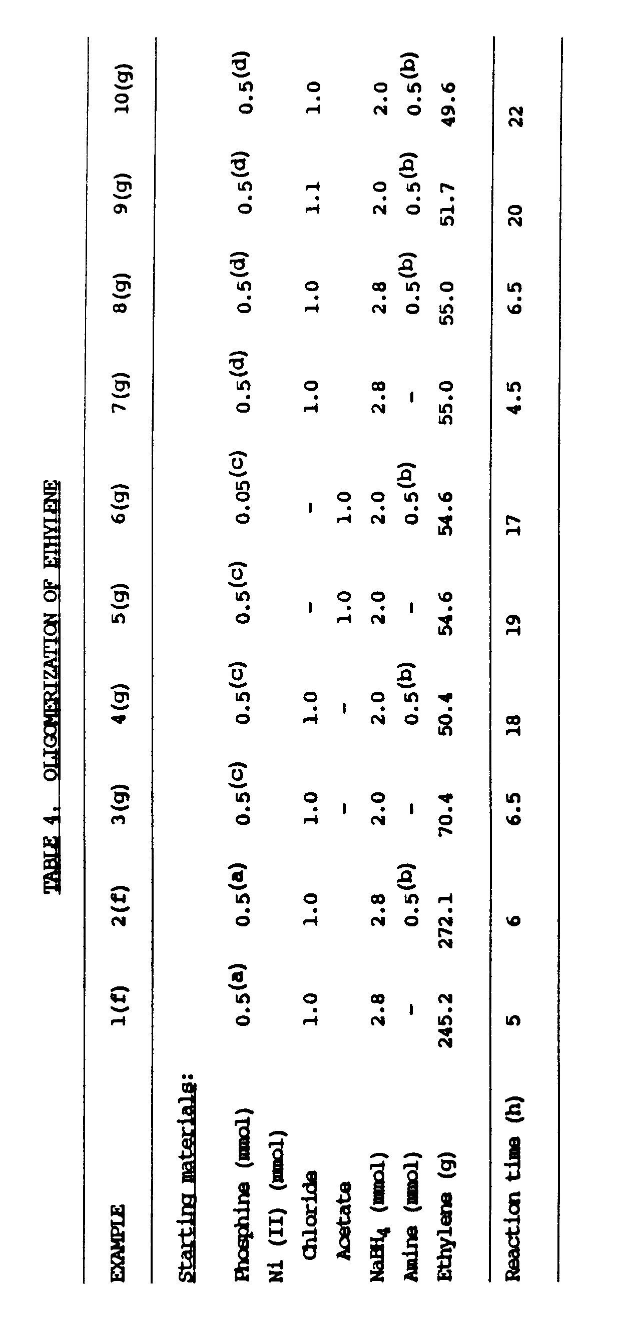

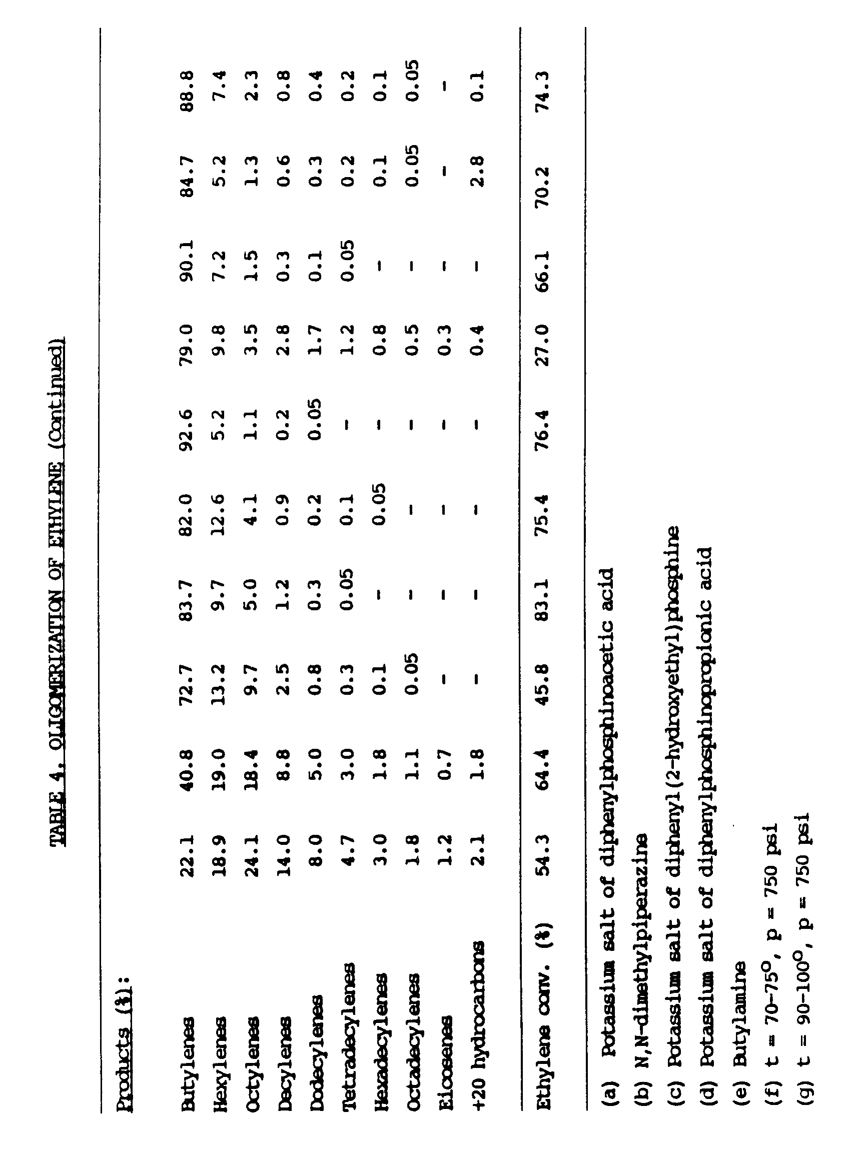

**Table 5**

| Typical product structure distribution in gas sample as obtained in the ethylene oligomerizing reaction. | |
|---|---|
| | % by weight |
| 2 Ethylene | 26.6 |
| 3 Ethane | 0.4 |
| 7 i + 1-butylene | 63.9 |
| 9 n-butane | 0.04 |
| 10 Tr-2-butylene | 2.1 |
| 12 Cis-2-butylene | 2.7 |
| 18 2-Et-1-butylene | 0.6 |
| 33 3-Me-1-pentene | 0.03 |
| 43 1-hexylene | 2.9 |
| 45 Tr-3-hexylene | 0.1 |
| 46 Cis-3-hexylene | 0.1 |
| 48 Tr-2-hexylene | 0.1 |
| 52 Cis-2-hexylene | 0.2 |
| 111 1-octylene | 0.03 |

## Claims

1. A process for manufacturing 1-butylene by selectively dimerizing ethylene, using as a catalyst a nickel chelate in which the ligand is diphenyl(carboxymethyl)-phosphine, diphenyl(2-carboxyethyl)-phosphine or their alkali metal salts or an alkali metal salt of diphenyl(2- hydroxyethyl)phosphine, in the presence of an amine derivative.

2. A process according claim 1 characterised in that the catalyst, either alone or with a promotor, is dissolved at 10-50°C in an organic solvent.

3. A process according to claim 1 or claim 2 characterized in that it is carried out at a temperature of 30-200°C, preferably 60-130°C, and under a pressure of 0.069-35 MPa, preferably 2.8-10 MPa.

4. A process according to claim 1 or claim 2, characterised in that the reaction time is 0.5 - 50 hours, preferably 3 - 30 hours.

5. A process according to any one of the preceding claims, characterized in that the molar proportions of nickel salt, ligand, reducing agent and promotor are 1-5:1:2-10:1-5, preferably 1.5-3:1:3-6:1-2.

6. A process according to any one of the preceding claims, characterized in that the nickel derivative used to prepare the catalyst is a nickel halide or nickel acetate, the acetate part serving as a promotor of the reaction.

7. A process according to any one of the preceding claims, characterized in that the reducing agent used to activate the reaction is a borohydride salt, preferably an alkali metal borohydride.

## Patentansprüche

1. Verfahren zur Herstellung von 1-Butylen durch selektive Dimerisation von Ethylen unter Verwendung eines Nickel-Chelats, in welchem der Ligand Diphenyl(carboxymethyl)phosphin, Diphenyl(2-carboxyethyl)phosphin oder deren Alkalimetallsalze oder ein Alkalimetallsalz von Diphenyl(2-hydroxyethyl)phosphin ist, als Katalysator in Gegenwart eines Aminderivats.

2. Verfahren nach Anspruch 1, dadurch gekennzeichnet, dass der Katalysator entweder allein oder mit einem Promotor bei 10-50°C in einem organischen Lösungsmittel gelöst wird.

3. Verfahren nach Anspruch 1 oder 2, dadurch gekennzeichnet, dass es bei einer Temperatur von 30-200°C, vorzugsweise von 60-130°C und unter einem Druck von 0,069-35 MPa, vorzugsweise von 2,8-10 MPa ausgeführt wird.

4. Verfahren nach Anspruch 1 oder 2, dadurch gekennzeichnet, dass die Reaktionsdauer 0,5-50 Stunden, vorzugsweise 3-30 Stunden beträgt.

5. Verfahren nach einem der vorangehenden Ansprüche, dadurch gekennzeichnet, dass die molaren Stoffmengenanteile von Nickelsalz, Ligand, Reduktionsagens und Promotor 1-5:1:2-10:1-5, vorzugsweise 1,5-3:1:3-6:1-2 betragen.

6. Verfahren nach einem der vorangehenden Ansprüche, dadurch gekennzeichnet, dass das zur Herstellung des Katalysators verwendete Nickelderivat ein Nickelhalid oder Nickelacetat ist, wobei der Acetat-Teil als Reaktionspromotor dient.

7. Verfahren nach einem der vorangehenden Ansprüche, dadurch gekennzeichnet, dass der zur Aktivation der Reaktion verwendete Reduktionsagens ein Borohydridsalz, vorzugsweise ein Alkalimetallborohydrid ist.

## Revendications

1. Procédé de préparation de 1-butylène par dimérisation sélective d'éthylène, utilisant comme catalyseur un chélate de nickel dans lequel le ligand est la diphényl(carboxyméthyl)phosphine, la diphényl(2-carboxyéthyl)phosphine ou leurs sels de métaux alcalins ou un sel de métal alcalin de la diphényl(2-hydroxyéthyl)phosphine, en présence d'un dérivé d'amine.

2. Procédé selon la revendication 1, caractérisé en ce que le catalyseur est dissous, soit seul, soit avec un promoteur, dans un solvant organique à 10-50°C.

3. Procédé selon la revendication 1 ou la revendication 2, caractérisé en ce qu'il est mis en oeuvre à une température de 30-200°C, de préférence de 60-130°C, et sous une pression de 0,069-35 MPa, de préférence de 2,8-10 MPa.

4. Procédé selon la revendication 1 ou la revendication 2, caractérisé en ce que le temps de réaction est de 0,5-50 heures, de préférence de 3-30 heures.

5. Procédé selon l'une quelconque des revendications précédentes, caractérisé en ce que les proportions molaires du sel de nickel, du ligand, de l'agent réducteur et du promoteur sont de 1-5:1:2-10:1-5, de préférence de 1,5-3:1:3-6:1-2.

6. Proce'dé selon l'une quelconque des revendications précédentes, caractérisé en ce que le dérivé de nickel utilisé pour la préparation du catalyseur est un halogénure de nickel ou l'acétate de nickel, la partie acétate servant de promoteur de la réaction.

7. Procédé selon l'une quelconque des revendications précédentes, caractérisé en ce que l'agent réducteur utilisé pour activer la réaction est un borohydrure, de préférence un borohydrure de métal alcalin.
